# EUROPEAN PATENT SPECIFICATION

(11) **EP 2 121 955 B1**
(45) Date of publication and mention of the grant of the patent: **28.12.2011**
(21) Application number: 07849174.3
(22) Date of filing: 19.11.2007
(51) Int. Cl.: C12P 19/34, C12Q 1/68

(54) **METHOD OF USE OF EPIREGULIN PROTEIN AND NUCLEIC ACID ENCODING THE SAME IN INFLAMMATORY CONDITIONS**
VERFAHREN ZUR VERWENDUNG VON EPIREGULIN-PROTEIN UND DAFÜR CODIERENDER NUKLEINSÄURE BEI ENTZÜNDUNGEN
PROCÉDÉ D'UTILISATION D'UNE PROTÉINE D'ÉPIRÉGULINE ET D'UN ACIDE NUCLÉIQUE CODANT POUR CELLE-CI DANS DES CONDITIONS INFLAMMATOIRES

(30) Priority: 20.11.2006 US 860139 P
(43) Date of publication of application: 25.11.2009
(73) Proprietor: Piramal Life Sciences Limited, Mumbai 400 013, Maharashtra (IN)
(72) Inventor: PADIGARU, Muralidhara, Mumbai 400 063 (IN); SHARMA, Somesh, Mumbai 400 063 (IN); THAKKAR, Arvind, Mumbai 400 063 (IN); RATHINA SABAPATHY, Anandharajan, Mumbai 400 063 (IN); PARIKH, Sapna Hasit, Mumbai 400 063 (IN); GHOSH, Usha, Mumbai 400 063 (IN)
(74) Representative: Richards, Michèle E.
(86) International application number: PCT/IB2007/054689
(87) International publication number: WO 2008/062359

(56) References cited:
- DRAPER BRADLEY K ET AL: "Topical epiregulin enhances repair of murine excisional wounds." WOUND REPAIR AND REGENERATION : OFFICIAL PUBLICATION OF THE WOUND HEALING SOCIETY [AND] THE EUROPEAN TISSUE REPAIR SOCIETY 2003 MAY-JUN, vol. 11, no. 3, May 2003 (2003-05), pages 188-197, XP002561151 ISSN: 1067-1927
- PANICCIA CHRISTINA ET AL: "Epiregulin increases the severity of cancer-predisposing colitis in the IL10 mouse model." PROCEEDINGS OF THE AMERICAN ASSOCIATION FOR CANCER RESEARCH ANNUAL MEETING, vol. 46, April 2005 (2005-04), page 462, XP001525106 & 96TH ANNUAL MEETING OF THE AMERICAN-ASSOCIATION-FOR-CANCER-RESEARCH; ANAHEIM, CA, USA; APRIL 16 -20, 2005 ISSN: 0197-016X
- BIÈCHE IVAN ET AL: "Molecular profiling of inflammatory breast cancer: identification of a poor-prognosis gene expression signature." CLINICAL CANCER RESEARCH : AN OFFICIAL JOURNAL OF THE AMERICAN ASSOCIATION FOR CANCER RESEARCH 15 OCT 2004, vol. 10, no. 20, 15 October 2004 (2004-10-15), pages 6789-6795, XP002561152 ISSN: 1078-0432
- SHIRAKATA ET AL: "Epiregulin, a member of the EGF family, is over-expressed in psoriatic epidermis" JOURNAL OF DERMATOLOGICAL SCIENCE, ELSEVIER SCIENCE PUBLISHERS, SHANNON, IR, vol. 45, no. 1, 20 September 2006 (2006-09-20), pages 69-72, XP005816345 ISSN: 0923-1811
- SHIRASAWA SENJI ET AL: "Dermatitis due to epiregulin deficiency and a critical role of epiregulin in immune-related responses of keratinocyte and macrophage." PROCEEDINGS OF THE NATIONAL ACADEMY OF SCIENCES OF THE UNITED STATES OF AMERICA 21 SEP 2004, vol. 101, no. 38, 21 September 2004 (2004-09-21), pages 13921-13926, XP002561153 ISSN: 0027-8424
- TOYODA H ET AL: "DISTRIBUTION OF MRNA FOR HUMAN EPIREGULIN, A DIFFERENTIALLY EXPRESSED MEMBER OF THE EPIDERMAL GROWTH FACTOR FAMILY" BIOCHEMICAL JOURNAL, THE BIOCHEMICAL SOCIETY, LONDON, GB, vol. 326, 1 January 1997 (1997-01-01), pages 69-75, XP009008473 ISSN: 0264-6021
- CONNOLLY, PH.: 'Effects of Exercise on Gene Expression in Human Peripheral Blood Mononuclear Cells.' J. APPL. PHYSIOL vol. 97, 2004, pages 1461 - 1469, XP008108964

## Description

### FIELD OF INVENTION

The present invention relates to nucleotide and protein sequences of epiregulin and methods employing them as a drug response marker in inflammatory conditions. The invention also provides epiregulin protein for use in the treatment of rheumatoid arthritis or juvenile rheumatoid arthritis.

### BACKGROUND OF THE INVENTION

Epiregulin is a member of epidermal growth factor (EGF) family of growth factors, which includes, among others, EGF, transforming growth factor (TGF)-alpha, amphiregulin (AR), heparin binding EGF, betacellulin, and the various heregulins. It was first identified in NIH 3T3 cell conditioned medium and is one of seven known ligands for the epidermal growth factor receptor (EGFR) (See Yarden et al., Nat. Rev. Mol. Cell Biol., 2001, 2:127-137). The coding sequence of human epiregulin cDNA predicts a 46-amino acid single-chain polypeptide, exhibiting 24-50% homology with the sequences of other EGFR-ligands.

Epiregulin is synthesized as a transmembrane precursor before being proteolytically cleaved to release a 46-amino-acid active protein. The mode of action of epiregulin is similar to other EGF family members in that it binds to and activates the tyrosine-kinase, ErbB family receptors (ErbB1 through B4). Unlike other EGFR ligands, epiregulin shows dual biological activity; it stimulates proliferation of fibroblasts, hepatocytes, smooth muscle cells, and keratinocytes but inhibits growth of several tumor-derived epithelial cell lines (*See* Toyoda et al., Biochem. J., 1997, 326:69-75). Epiregulin also shows more potent bioactivity in vitro than other EGF-like growth factors. EGF family members, including epiregulin, play a fundamental role in epithelial tissues and human keratinocytes in an autocrine manner; however, their roles in immune responses and the physiological role of epiregulin remain to be elucidated.

Epiregulin is one of the ligands for the epidermal growth factor receptor (EGFR). The EGF superfamily and EGFR family, including EGFR (ErbB1), ErbB2 (HER2), ErbB3 (HER3), and ErbB4 (HER4) (*See* Riese & Stern, BioEssays 1998, 20:41-48), play a fundamental role in epithelial tissues. EGF family members, including EGF, transforming growth factor-α, amphiregulin, heparin binding EGF (HB-EGF), epiregulin, and other members, regulate these receptors by inducing their homo- and or hetero-oligomerization (*See* Yarden & Sliwkowski, Nat. Rev. Mol. Cell Biol., 2001, 2:127-137). EGF family members vary in their ability to activate distinct ErbB heterodimers, and this mechanism may, in part, account for the differences in their bioactivities (*See* Jackson et al., EMBO J., 2003, 22:2704-2716)

The membrane-anchored precursor of the EGF family is enzymatically processed externally to release a mature soluble form that acts as autocrine and or paracrine growth factor, whereas some members of the EGF family act in the membrane-anchored form. A bioactive transmembrane precursor, pro-HB-EGF, was suggested to induce growth inhibition or apoptosis rather than the proliferative response induced by soluble HB-EGF. Epiregulin acts as an autocrine growth factor in normal human keratinocytes *in vitro,* in the differentiation of vascular smooth muscle cells and as one of the paracrine mediators that propagate luteinizing hormone signals in the ovarian follicle; however, its physiological role *in vivo* still remains unknown.

Both the membrane-bound and secreted forms of epiregulin are identified. Sirasawa et al (*See* PNAS, 2004, 101:13921) have reported that epiregulin deficiency causes chronic dermatitis in mice.

The inflammatory response represents the body's response to injury which can arise from a variety of agents such as infectious organisms, toxins including alcohol, allergies, autoimmune responses, trauma, lacerations, burns, circulatory occlusions and other factors that induce tissue damage. At present, C-reactive protein is used extensively as marker of inflammatory status. However, it is a non-specific marker that cannot diagnose a particular disease but is used to gain insight into conditions of inflammation. More sensitive markers are needed either as individual markers or used in combination with C-reactive protein for greater prognostic value (Philips et al., J.of Endocrinology, 1999; 161, 195-198).

Wound healing is the process of repair that follows injury to the skin and other soft tissues. The process of wound healing involves two essential components - regeneration and repair. In regeneration, a specialized tissue is replaced by the proliferation of surrounding undamaged specialized cells. In repair, lost tissue is replaced by granulation tissue to form scar tissue.

Psoriasis (PS) is a common chronic skin disease characterized by skin inflammation and hyperproliferation of epidermal keratinocytes that can be triggered by both genetic and environmental risk factors It affects approximately 2% of individuals from the northern European population. The three cardinal features of psoriatic lesions are erythema, indurations and scaling. An association with psoriatic arthritis is seen in 6-10% of cases. In young patients it is frequently triggered by infection with group A streptococci, and can also occur with human immunodeficiency virus (HIV) infection (See Duvic et al., J. Invest. Dermatol., 1990, 95:38S-40S). The pathogenesis of psoriasis remains to be elucidated.

Rheumatoid arthritis (RA) is a systemic disease with highly variable clinical manifestations. Rheumatoid arthritis generally affects the joints of the extremities and involves synovial joints between two bones. It is a chronic inflammatory disease that goes through several stages and finally results in a massive destruction of joints or inflammations in the tendon area. According to the recent state of knowledge, it is considered that T cells initiate and maintain the inflammation. In this process, cytokines as well as mesenchymal cells (macrophages and synovial fibroblasts) are active. It is very probable that the cytokine TNF-α is one of the mediators of the inflammation. It promotes the formation of the pannus, which is typical of RA, and promotes cartilage destruction. TNF-α increases the number of adhesion molecules for leukocytes on the surface of the endothelial cells and the penetration of the capillary endothelial layer. This results in an increased inflow of leukocytes into the synovia. The cytokine promotes the secretion of matrix metalloproteinases by the synoviocytes. These enzymes are directly involved in the destruction of bones and cartilage. TNF-α also sensitizes pain receptors, which is associated with the induction of pain sensations. TNF-α plays a critical role in the initiation and maintenance of RA.

Changes in gene and/or protein expression have been described previously in psoriasis and other inflammatory diseases. IL-1 and TNF-α are up-regulated in psoriasis and activate several cellular signaling pathways including the NF-κB pathway. Differential gene expression of many transcripts has been reported in psoriatic skin as compared to the normal skin (See Bowcock et al, Human Molecular Genetics, 2001, 10:1793). The modification could be either up-regulation (e.g.: IL-8R) or down-regulation (e.g.: cysteinc-rich protein 1).

Draper Bradley K. et al., "Topical epiregulin enhances repair of murine excisional wounds", Wound Repair and Regeneration : Official Publication of the Wound Healing Society [and] the European Tissue Repair Society, 2003 May-Jun, vol. 11, no.3, May 2003 (2003-05), pages 188-197, discloses that topical epiregulin enhances the repair of murine excisional wounds, in particular epidermal and dermal proliferation and thickening.

Paniccia Christina et al., ``Epiregulin increases the severity of cancer-predisposing colitis in the IL10 mouse model", Proceedings of the American Association for Cancer Research Annual Meeting, vol. 46, April 2005 (2005-04), page 462, XP001525106, discloses that a dextran sulfate sodium chemical model of colitis in interleukin-10 deficient mice shows epiregulin to be required for normal intestinal homeostasis and protection from chemically-induced colitis, whereas double mutant mice deficient for epiregulin and interleukin-10 show reduced severity of inflammation-related intestinal damage.

### SUMMARY OF THE INVENTION

The present invention relates to nucleotide and protein sequences of epiregulin and their use as a drug response marker in inflammatory conditions. Moreover the invention relates to epiregulin protein for use in the treatment of rheumatoid arthritis and juvenile rheumatoid arthritis.

In one aspect the invention provides a method for predicting the response of a patient to a drug employed in the treatment of an inflammatory condition in the patient, the method comprising: determining the level of epiregulin gene expression in the patient's blood sample; and predicting the response of the patient to the drug employed in the treatment of the inflammatory condition.

In another aspect, the invention provides a method for monitoring drug response in a patient receiving treatment for an inflammatory condition, the method comprising: determining the level of epiregulin protein in the patient's serum sample; and monitoring drug response in the patient receiving treatment for an inflammatory condition.

In yet another aspect, the invention provides a method of determining the likelihood of a subject developing an inflammatory condition, the method comprising: determining the level of epiregulin gene expression in the subject's blood sample.

In another aspect the invention provides a method of monitoring drug response in a patient receiving treatment for an inflammatory condition with an anti-inflammatory compound, the method comprising: measuring epiregulin gene expression in the patient's blood or serum sample for elevated levels of the epiregulin gene expression; and monitoring drug response in the patient receiving treatment for the inflammatory condition with the anti-inflammatory compound.

Another aspect of the invention provides epiregulin protein, alone or in combination with at least one anti-inflammatory compound, for use in the treatment of rheumatoid arthritis or juvenile rheumatoid arthritis.

### DETAILED DESCRIPTION OF THE INVENTION

A method of detecting an increase in epiregulin gene expression following administration of anti-inflammatory agent is described. Also described is the use of epiregulin as a drug response marker for inflammatory conditions, and the use of epiregulin protein levels to diagnose an inflammatory condition in a subject. It is described herein that epiregulin mitogen like growth factor reacts with EGFR and is involved in epithelial cell proliferation and other biological processes. Furthermore, the inventors' studies show that secreted epiregulin is selectively up regulated in response to anti-inflammatory agent, which clearly indicates its use as a drug response marker. In addition, epiregulin regulates wound healing by down-regulation of inflammatory processes and epithelial cell proliferation in keratinocytes, which is indicative of its therapeutic potential for inflammatory conditions like psoriasis.

The length of epiregulin polynucleotide is 506 bp, the accession no. is NM_001432, and the length of protein sequence is 169 amino acids. Epiregulin gene of the present invention has a DNA sequence, encoded by polynucleotide as shown in Table 1 and protein sequence as in Table 2.

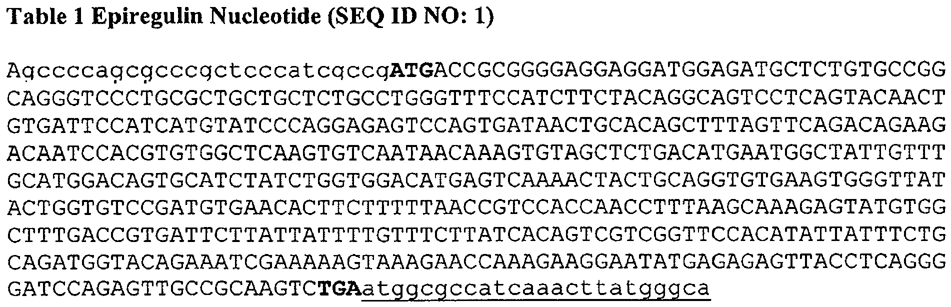

**Table 2 Epiregulin Protein (SEQ ID NO: 2)**

| |
|---|
| Human Epiregulin protein sequence (NP_001423, HUMAN_EREG) 169 amino acids |
| MTAGRRMEMLCAGRVPALLLCLGFHLLQAVLSTTVIPSCIPGESSDNCTALVQTEDNPRVAQVS ITKCSSDMNGYCLHGQCIYLVDMSQNYCRCEVGYTGVRCEHFFLTVHQPLSKEYVALTVILIIL FLITVVGSTYYFCRWYRNRKSKEPKKEYERVTSGDPELPQV |

The inventors' studies employed a method of first isolating RNA from normal human peripheral blood leukocytes treated with anti-inflammatory drugs (such as those disclosed in published PCT patent application WO2006051477) and Rolipram. (Rolipram is used as a positive control drug to identify potential therapeutic agents for the treatment of chronic inflammatory disorders such as rheumatoid arthritis.) The isolate leukocytes were activated with bacterial lipopolysaccharide. The quality and quantity of RNA was measured by spectrometry and gel electrophoresis. Gene expression profiling was performed by microarray procedure that is known in the art.

Increased expression of epiregulin in response to anti-inflammatory compound indicates that the polynucleotides and polypeptides of the present invention are useful as drug response markers. The marker can be employed for differential identification of responders versus non-responders to treatment of, for example, inflammation and inflammatory disorders. Epiregulin, which is a secreted protein, can be detected in the blood sample of the subject in measurable quantities. Elevated epiregulin levels in the sample arc indicative of positive response to anti-inflammatory compounds.

The polynucleotides and polypeptides of the invention are also useful as diagnostic markers for identification of the disease condition in a given biological sample in conditions that include, but are not limited to, inflammation and inflammatory disorders. Epiregulin can be detected in the blood or serum sample of the subject in measurable quantities. Modified epiregulin levels in the subject's sample are indicative of susceptibility to inflammation or inflammatory conditions.

The present invention includes epiregulin protein, alone or in combination with at least one anti-inflammatory compound, for use in the treatment of rheumatoid arthritis or juvenile rheumatoid arthritis. Known anti-inflammatory compounds such as cyclophosphamide and azathioprine, dexamethasone and hydrocortisone, may be used in combination with epiregulin protein. The two compounds, epiregulin and the anti-inflammatory agent, can be administered once or twice daily as required. The compounds can be administered simultaneously or sequentially. Epiregulin may be formulated with an anti-inflammatory compound and administered to the patient that needs treatment for an inflammatory condition. It is believed that administering epiregulin as a treatment for psoriasis may be based on epiregulin induced epithelial cell proliferation and down regulation of inflammatory processes in the skin. These two features promote wound healing.

By use of the methods of the invention, one skilled in the genomics art can identify multiple groups of related genes, many representing processes with previously unrecognized relationships to immune related or inflammatory conditions. Compositions of matter comprising sets of genes, polynucleotides, polypeptides encoded by said polynucleotides, may be identified as being involved in treating skin diseases, cancer, and inflammatory conditions. In particular, the set of genes can be used for the treatment of inflammatory conditions such as psoriasis and arthritis.

### Definitions

Biomarker is a well-known and useful concept in the genomic and biopharmaceutical art. "Biomarker" referred to herein, is a measurable biological manifestation that is a quantitative indication of the presence and degree of an underlying biological process of interest e.g.: drug response or diagnosis.

As used herein, a "polynucleotide" refers to a molecule having a nucleic acid sequence contained in SEQ ID NO: 1. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence.

Moreover, as used herein, a "polypeptide" refers to a molecule having the translated amino acid sequence generated from the polynucleotide as broadly defined. The polypeptide used in the present invention is exemplified by SEQ ID NO: 2.

As used herein, a "secreted" protein refers to those proteins capable of being directed to the endoplasmic reticulum, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

A polypeptide having "biological" activity refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention, for example, the candidate polypeptide may exhibit greater activity or not more than about 25-fold less and, preferably, not more than about tenfold less activity, and most preferably, not more than about three-fold less activity relative to the polypeptide of the present invention.

The invention is further illustrated in the following non-limiting examples

### Examples:

Compound P979 was synthesized according to processes described in published PCT patent application WO2006051477. P979 is an anti-inflammatory compound demonstrated to have an IC₅₀ less than 1 µM as reported in Example 43, Table 2 of the PCT application WO2006051477.

### Example 1: Epiregulin expression in peripheral blood monocytes (PBMNCs) after exposure to anti-inflammatory agent and its study by microarray based expression profiling

### Method:

Exposure of PBMCs to P979 and Rolipram: Human peripheral blood leukocytes were obtained from normal blood donors by density gradient separation, washed with PBS (phosphate buffered saline), and plated on petri dishes using RPMI medium. Cells were treated for 30 minutes with test compounds at concentrations 0.3, 1.0 and 3.0 µM and with Rolipram at 300 µM as a control as shown in the results section and subsequently activated with bacterial lipopolysaccharide (LPS). After 2 hours, cells were harvested and RNA was prepared.

Preparation of RNA: RNA was prepared by adding 1 ml of trizol reagent (Sigma) subsequently purified using Qiagen-RNAEasy kit, using the instruction from manufacturer's protocol. Quality and quantity of RNA was measured by spectrometry and gel electrophoresis.

Gene expression profiling by microarray: A total of 10 µg of RNA was used to generate cRNA, which is subsequently labeled with Cy3 or Cy5 fluorescent dyes. Control and test cRNAs labeled with Cy3 and Cy5 respectively were pooled and hybridized to DNA chip. DNA chips were synthesized in house and contains 23, 290 oligomers (70-mer oligonucleotides, obtained from Illumina, U.S.A.) that represent well-annotated reference gene list from human genome. Pronto plus microarray kit from Promega was used throughout the microarray procedure according to the manufacturer's protocol. Hybridized DNA chips were scanned and images were generated. Following image analysis, image intensity for each of the 23, 290 spots were measured for test and control slides.

### Results:

Gene expression was expressed as a ratio of the image intensity between test and control slide for a given gene (Table 3). Ratio of intensity of one between normal and test slide was considered as normal, whereas ratio of 0.5 and less was considered as down-regulated expression. A ratio of 1.5 and above was considered as gene up-regulation.

**Table 3: EREG expression in PBMCs after exposure to anti-inflammatory agent as studied by microarray based expression profiling**

| | P979 | | | Rolipram |
|---|---|---|---|---|
| Drug concentration | 0.3 µM | 1.0 µM | 3.0 µM | 300 µM |
| Ratio intensity | 1.42 | 2.63 | 2.95 | 1.03 |

As shown in the above data, the expression of epiregulin gene is up-regulated selectively by anti-inflammatory agents in a concentration dependant manner, but not by the control compound Rolipram.

### Confirmation of data obtained from Microarray:

Real Time Polymerase Chain Reaction (RT-PCR): To confirm the data obtained from microarray, we performed RT-PCR using specific primers for epiregulin gene.

EREG expression in PBMCs after exposure to anti-inflammatory agent was confirmed by real time polymerase chain reaction (RT-PCR). Microarray based gene expression profiling studied large-scale gene expression. RT PCR focused on the gene/genes of interest and expression analysis was carried out under similar experimental conditions. Left primer CGTGTGGCTCAAGTGTCAAT;
Right primer TGGAACCGACGACTGTGATA;
Product size: 235 base pairs.

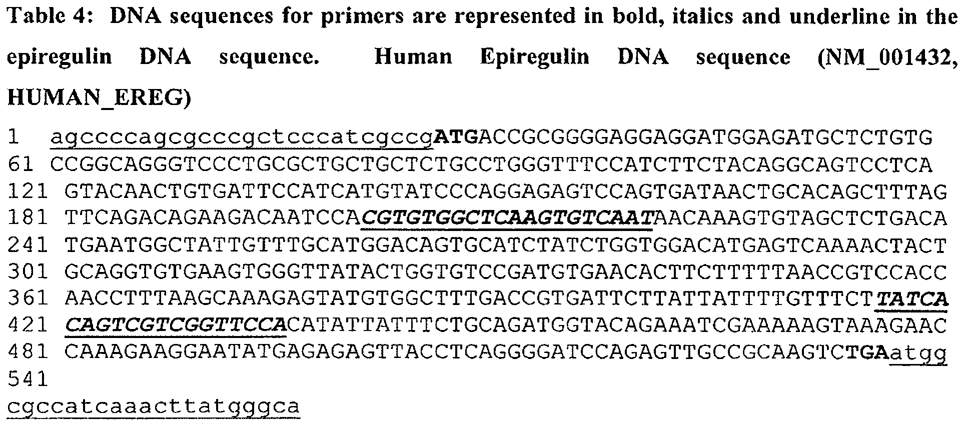

The aliquot of RNA samples as used for microarray study was used for RT-PCR analysis. The experiment was carried out using cMaster^{™} RTplusPCR System kit from Eppendorf using the protocol as provided in the kit. Briefly, cDNA was synthesized using RNA from various sources as shown in the results and subsequently quantified during amplification step by incorporation of Syber green dye into the amplicons.

The RT-PCR analysis confirmed the finding of specific activation of EREG in response to P979 at certain drug concentrations (Table 5)

**Table 5 - EREG Expression in PBMC following treatment with P979 and Rolipram**

| Drug Used | Concentration (µM) | Log-fold Change |
|---|---|---|
| P979 | 0.3 | 0.93 |
| | 0.3 | 1.3 |
| | 1 | 3.4 |
| | 3 | 3.2 |
| | 3 | 2.0 |
| Rolipram | 10 | 1.2 |

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

All publications and patent applications in this specification are indicative of the level of ordinary skill in the art to which this invention pertains.

The invention has been described with reference to various specific and preferred embodiments and techniques. However, it should be understood that many variations and modifications may be made while remaining within the scope of the invention.

## Claims

1. A method for predicting the response of a patient to a drug employed in the treatment of an inflammatory condition in the patient, the method comprising: determining the level of epiregulin gene expression in the patient's blood sample; and predicting the response of the patient to the drug employed in the treatment of the inflammatory condition.

2. The method of claim 1, wherein the level of the epiregulin gene expression is elevated in the patient's blood sample.

3. The method of claim 1 or claim 2, wherein the inflammatory condition comprises rheumatoid arthritis, psoriasis, atopic dermatitis, psoriatic arthritis, juvenile rheumatoid arthritis, or a combination thereof.

4. A method for monitoring drug response in a patient receiving treatment for an inflammatory condition, the method comprising: determining the level of epiregulin protein in the patient's serum sample; and monitoring drug response in the patient receiving treatment for an inflammatory condition.

5. The method of claim 4, wherein the level of the epiregulin protein is elevated in the patient's serum sample.

6. The method of claim 4 or claim 5, wherein the inflammatory condition comprises rheumatoid arthritis, psoriasis, atopic dermatitis, psoriatic arthritis, juvenile rheumatoid arthritis, or a combination thereof.

7. A method of determining the likelihood of a subject developing an inflammatory condition, the method comprising: determining the level of epiregulin gene expression in the subject's blood sample.

8. Use of epiregulin protein alone or in combination with at least one anti-inflammatory compound in the manufacture of a medicament for the treatment of rheumatoid arthritis or juvenile rheumatoid arthritis.

9. The use of claim 8, wherein the epiregulin is administered as an oral, parenteral, subcutaneous or topical formulation.

10. A method of monitoring drug response in a patient receiving treatment for an inflammatory condition with an anti-inflammatory compound, the method comprising: measuring epiregulin gene expression in the patient's blood or serum sample for elevated levels of the epiregulin gene expression; and monitoring drug response in the patient receiving treatment for the inflammatory condition with the anti-inflammatory compound.

11. Epiregulin protein for use in the treatment of rheumatoid arthritis or juvenile rheumatoid arthritis.

12. Epiregulin protein in combination with at least one anti-inflammatory compound for use in the treatment of rheumatoid arthritis or juvenile rheumatoid arthritis.

13. Epiregulin protein of claim 11 or combination of claim 12, in a form suitable for oral, parenteral, subcutaneous or topical administration.

## Patentansprüche

1. Ein Verfahren zum Prognostizieren des Ansprechens eines Patienten auf ein Medikament, das in der Behandlung eines entzündlichen Zustands bei einem Patienten genutzt wird, wobei das Verfahren umfasst: Bestimmen des Levels der Epiregulin-Genexpression in der Blutprobe des Patienten; und Prognostizieren des Ansprechens des Patienten auf das Medikament, das in der Behandlung des entzündlichen Zustands genutzt wird.

2. Das Verfahren nach Anspruch 1, wobei der Level der Epiregulin-Genexpression in der Blutprobe des Patienten erhöht ist.

3. Das Verfahren nach Anspruch 1 oder 2, wobei der entzündliche Zustand rheumatoide Arthritis, Psoriasis, atopische Dermatitis, Psoriasisarthritis, juvenile rheumatoide Arthritis oder eine Kombination davon umfasst.

4. Ein Verfahren zur Überwachung der Ansprache eines Patienten, der eine Behandlung gegen einen entzündlichen Zustand bekommt, auf ein Medikament, wobei das Verfahren umfasst: Bestimmen des Levels von Epiregulin-Protein in der Serumprobe des Patienten; und Überwachen der Ansprache des Patienten, der eine Behandlung gegen einen entzündlichen Zustand bekommt, auf ein Medikament.

5. Das Verfahren nach Anspruch 4, wobei der Level des Epiregulin-Proteins in der Serumprobe des Patienten erhöht ist.

6. Das Verfahren nach Anspruch 4 oder 5, wobei der entzündliche Zustand rheumatoide Arthritis, Psoriasis, atopische Dermatitis, Psoriasisarthritis, juvenile rheumatoide Arthritis oder eine Kombination davon umfasst.

7. Ein Verfahren des Bestimmens der Wahrscheinlichkeit für einen Probanden, einen entzündlichen Zustand zu entwickeln, wobei das Verfahren umfasst:
Bestimmen des Levels der Epiregulin-Genexpression in der Blutprobe des Probanden.

8. Gebrauch des Epiregulin-Proteins allein oder in Kombination mit wenigstens einem entzündungshemmenden Präparat in der Herstellung eines Medikaments für die Behandlung rheumatoider Arthritis oder juveniler rheumatoider Arthritis.

9. Der Gebrauch nach Anspruch 8, wobei das Epiregulin als orale, parenterale, subkutane oder topische Zubereitung verabreicht wird.

10. Ein Verfahren des Überwachens der Ansprache eines Patienten, der eine Behandlung eines entzündlichen Zustandes mit einem entzündungshemmenden Präparat bekommt, auf ein Medikament, wobei das Verfahren umfasst: Messen der Epiregulin-Genexpression in der Blut- oder Serumprobe des Patienten auf erhöhte Level der Epiregulin-Genexpression; und Überwachen der Ansprache des Patienten, der eine Behandlung eines entzündlichen Zustandes mit einem entzündungshemmenden Präparat bekommt, auf ein Medikament.

11. Epiregulin-Protein für den Gebrauch in der Behandlung von rheumatoider Arthritis oder juveniler rheumatoider Arthritis.

12. Epiregulin-Protein in Kombination mit wenigstens einem entzündungshemmenden Präparat für den Gebrauch in der Behandlung von rheumatoider Arthritis oder juveniler rheumatoider Arthritis.

13. Epiregulin-Protein nach Anspruch 11 oder Kombination nach Anspruch 12, in einer für orale, parenterale, subkutane oder topische Verabreichung passenden Form.

## Revendications

1. Procédé permettant de prédire la réponse d'un patient à un médicament employé dans le traitement d'une affection inflammatoire chez le patient, le procédé comprenant : la détermination du taux d'expression du gène de l'épiréguline dans l'échantillon de sang du patient ; et de prédire la réponse du patient au médicament employé dans le traitement de l'affection inflammatoire.

2. Procédé selon la revendication 1, dans lequel le taux d'expression du gène de l'épiréguline est élevé dans l'échantillon de sang du patient.

3. Procédé selon la revendication 1 ou la revendication 2, dans lequel l'affection inflammatoire comprend la polyarthrite rhumatoïde, le psoriasis, la dermite atopique, l'arthrite psoriasique, la polyarthrite rhumatoïde juvénile ou une combinaison de ceux-ci.

4. Procédé permettant de contrôler la réponse à un médicament chez un patient recevant un traitement pour une affection inflammatoire, le procédé comprenant : la détermination du taux de la protéine épiréguline dans l'échantillon de sérum du patient ; et de contrôler la réponse au médicament chez le patient recevant le traitement pour une affection inflammatoire.

5. Procédé selon la revendication 4, dans lequel le taux de la protéine épiréguline est élevé dans l'échantillon de sérum du patient.

6. Procédé selon la revendication 4 ou la revendication 5, dans lequel l'affection inflammatoire comprend la polyarthrite rhumatoïde, le psoriasis, la dermite atopique, l'arthrite psoriasique, la polyarthrite rhumatoïde juvénile ou une combinaison de ceux-ci.

7. Procédé permettant de déterminer la probabilité d'un sujet de développer une affection inflammatoire, le procédé comprenant : la détermination du taux d'expression du gène de l'épiréguline dans l'échantillon de sang du sujet.

8. Utilisation de la protéine épiréguline seule ou en combinaison avec au moins un composé anti-inflammatoire dans la fabrication d'un médicament destiné au traitement de la polyarthrite rhumatoïde ou de la polyarthrite rhumatoïde juvénile.

9. Utilisation selon la revendication 8, où l'épiréguline est administrée sous la forme d'une formulation orale, parentérale, sous-cutanée ou topique.

10. Procédé permettant de contrôler la réponse à un médicament chez un patient recevant un traitement pour une affection inflammatoire avec un composé anti-inflammatoire, le procédé comprenant : la mesure de l'expression du gène de l'épiréguline dans l'échantillon de sang ou de sérum du patient à la recherche de taux élevés de l'expression du gène de l'épiréguline ; et de contrôler la réponse à un médicament chez le patient recevant un traitement pour l'affection inflammatoire avec le composé anti-inflammatoire.

11. Protéine épiréguline pour une utilisation dans le traitement de la polyarthrite rhumatoïde ou de la polyarthrite rhumatoïde juvénile.

12. Protéine épiréguline en combinaison avec au moins un composé anti-inflammatoire pour une utilisation dans le traitement de la polyarthrite rhumatoïde ou de la polyarthrite rhumatoïde juvénile.

13. Protéine épiréguline selon la revendication 11 ou combinaison selon la revendication 12, sous une forme appropriée pour une administration orale, parentérale, sous-cutanée ou topique.
